Europäisches Patentamt

European Patent Office

Office européen des brevets

(19)

(11) Veröffentlichungsnummer: **0 151 989 B1**

# (12) EUROPÄISCHE PATENTSCHRIFT

(45) Veröffentlichungstag der Patentschrift: **10.04.91**

(51) Int. Cl.⁵: **A61K 31/355**, A61K 9/48, A61K 31/44

(21) Anmeldenummer: **85100752.6**

(22) Anmeldetag: **25.01.85**

Teilanmeldung 89113512.1 eingereicht am 25/01/85.

Die Akte enthält technische Angaben, die nach dem Eingang der Anmeldung eingereicht wurden und die nicht in dieser Patentschrift enthalten sind.

(54) **Mittel zur Behandlung von Herzerkrankungen.**

(30) Priorität: **28.01.84 DE 3402928**
**15.02.84 DE 3405239**
**27.02.84 DE 3407025**
**04.06.84 DE 3420738**

(43) Veröffentlichungstag der Anmeldung:
**21.08.85 Patentblatt 85/34**

(45) Bekanntmachung des Hinweises auf die Patenterteilung:
**10.04.91 Patentblatt 91/15**

(84) Benannte Vertragsstaaten:
**AT BE CH DE FR GB IT LI LU NL SE**

(56) Entgegenhaltungen:
**DE-A- 2 425 222**
**FR-A- 2 143 564**
**US-A- 3 932 634**

**ROTE LISTE, 1983, Editio Cantor, Aulendorf/Württ., DE, Nr. 31150: "Delta Pima-fucort"**

(73) Patentinhaber: **Ismail, Roshdy, Dr.**

**Siebengebirgs-Apotheke Siebengebirgsallee 2**
**W-5000 Köln 41 (Klettenberg)(DE)**

(72) Erfinder: **Ismail, Roshdy, Dr.**
**Siebengebirgs-Apotheke Siebengebirgsallee 2**
**W-5000 Köln 41 (Klettenberg)(DE)**

(74) Vertreter: **Werner, Hans-Karsten, Dr. et al**
**Deichmannhaus am Hauptbahnhof**
**W-5000 Köln 1(DE)**

Rank Xerox (UK) Business Services

EP 0 151 989 B1

B. HELWIG: "Moderne Arzneimittel", 5. Auflage, 1980, Wissenschaftliche Verlagsgesellschaft, Stuttgart, DE

ROTE LISTE, 1981, "Salus Herz-Schutz-Kapseln"

ROTE LISTE, 1981, "Lani Longural"

ROTE LISTE, 1983, "Eusovit 300", "E-Vicotrat", "Evion"

ROTE LISTE, 1983, "FegaCoren N pro inj."

LENZHOFER R. et al.: "Acute cardiac toxicity in patients after doxorubicin treatment and the effect of combined tocopherol and nifedipine", Journal of Cancer Res. Clin. Oncol. 106 (2), 143-7 (1983).

## Beschreibung

Die vorliegende Erfindung betrifft eine neue Verwendung von Vitamin E im Bereich der Verstärkung und Verbesserung der Wirkung oral verabreichten Nifedipins und in Mitteln zur Behandlung von Herzerkrankungen, die Nifedipin enthalten und oral verabreicht werden.

Vitamin E ist bekannt als Antioxidans und Schutzvitamin für Phospholipide der Zellmembran. Es hält die Permeabilität und Stabilität der Zellmembran aufrecht; Lucy, Ann. N.Y. Academy of Science 203 1972, S. 4. Es ist weiterhin bekannt, daß Vitamin E eine membranabdichtende Wirkung besitzt; F. Mittelbach und G. Bodechtel, Münchner Medizinische Wochenschrift 110 (1968) 36: 1988-1993. Bei Erythrocyten, den einfachsten Zellen des menschlichen Körpers, wurde festgestellt, daß Vitamin E eine Schutzwirkung für die Zellmembran hat. In Tierversuchen und beim Menschen wurde bewiesen, daß Anämie das erste Anzeichen von Vitamin-E-Mangel ist. Bei Gabe von hohen Vitamin-E-Dosen normalisiert sich die Hämolyse der Erythrocyten; vgl. William J. Darbey Vitamin Horm, 26 (50) S. 685-704 (1968) und Phelps DL Pediatrics 63 (6) S. 933-935 (1979). Aus diesen Literaturstellen geht hervor, daß bei Gabe von 200 bis 800 mg Vitamin E oral für einen Zeitraum von 1 bis 4 Tagen, die Hämolyse der Erythrocyten significant verbessert wird im Vergleich zu Patienten mit Vitamin-E-Mangel.

Weiterhin ist bekannt, daß 750 mg Vitamin E täglich in einem Zeitraum von 3 bis 6 Monaten erfolgreich bei Thalassamie-Patienten eingesetzt wurde, wobei eine Nor malisierung der Hämolyse der Erythrocyten beobachtet wurde; vgl. Kahane I. ISR. J. med. 12 (1), S. 11-15 (1976).

Erfolgreich eingesetzt wurde Vitamin E weiterhin bei Patienten mit akuter Hepatitis und alkoholischer Hepatitis, die einen Mangel an Vitamin E im Serum haben; vgl. Yoshiakawa T. Takemura S. Kato H. et al. Japan J. Gastrovent, 74/7, S. 732-739 (1977). Schließlich wurde Vitamin E bei Patienten mit Eisenmangelanämie eingesetzt und bewirkte während eines Zeitraumes von 4 bis 8 Wochen eine Verbesserung bzw. Normalisierung des Lipidmetabolismus im Knochenmark; vgl. Takoshi Itaga, Central Clinical Laboratory Nagasaki University of Medicine, Japan.

Genauere Untersuchungen bezüglich der Resorption und der Wirksamkeit von Vitamin E haben ergeben, daß ein Großteil des Vitamin E durch die Magensäure zerstört wird, so daß nur ein Teil des Vitamin E im Körper zur Wirksamkeit kommen kann; vgl. Arthur Vogelsang in Angiology 21, S. 275-79 (1970).

Aus Arzneimittel-Forschung 24, Nr. 2 (1974), 202-205, und 21, Nr. 3 (1971), 335-342, ist bekannt, daß mit Hilfe von Vitamin E eine wesentliche Toleranzsteigerung herzwirksamer Glycoside erzielt werden kann. Ein Zusammenhang zwischen Wirkung des Vitamin E und Vitamin-E-Dosis wurde jedoch ausdrücklich verneint.

In der Roten Liste 1981 (Editio Cantor, Aulendorf/ Württ.) werden zur Vorbeugung gegen u. a. Herz-Kreislaufstörungen Kapseln offenbart, die mindestens 1 mg Flavonglykoside neben 200 I. E. D-alpha-Tocopherolacetat und weiteren Komponenten enthalten.

In "Moderne Arzneimittel, 5. Auflage, Wiss. Verlagsgesellschaft, Stuttgart (1980), Seite 954" wird ein Präparat "Echtrovit" in Kapselform offenbart, das 30 mg Vitamin E-acetat und 10 mg Rutin neben weiteren Komponenten enthält und u. a. gegen Durchblutungsstörungen und Kreislaufschwäche verabreicht werden soll.

In der DE-A 2 425 222 wird unter anderem die Verwendung einer Kombination von Vitamin E mit Vincamin und gegebenenfalls auch Troxerutin in einem Mittel zur Bekämpfung von Myokardinfarkt, Coronaritis, Arteritis, Arteriosklerose, thrombo-embolischen Krankheiten und Kreislaufschwierigkeiten beschrieben, das u. a. auch oral verabreicht werden kann.

Die in den genannten Dokumenten beschriebenen Kombinationen vasoaktiver Wirkstoffe mit Vitamin E wirken auf dem Sektor Herz-/Kreislaufkrankheiten, doch sind die Mechanismen ihrer physiologischen Wirkung nicht nur voneinander völlig verschieden, sondern unterscheiden sich auch fundamental von der Wirkungsweise des Nifedipins im Organismus.

In der US-A 3,784,684 werden oral verabreichbare Arzneimittel, beispielsweise Kapseln, angegeben, die als coronar wirksame Substanz 1,4-Dihydro-2,6-dimethyl-4-(2'-nitrophenyl-) pyridin-3,5-dicarbonsäure-dimethylester (Nifedipin) enthalten. Eine Kombination dieses Wirkstoffs mit Vitamin E wird weder offenbart noch nahegelegt.

Es wurde jetzt gefunden, daß Vitamin E überraschenderweise geeignet ist, die Wirkung von 1,4-Dihydro-2,6-dimethyl-4-(2'-nitrophenyl-)pyridin-3,5-dicarbonsäuredimethylester (Nifedipin) zu verstärken und zu verbessern. Weiterhin fördert Nifedipin die Wirksamkeit von Vitamin E.

Dieser neue Indikationsbereich war aufgrund des bisherigen Wissenstandes nicht vorherzusehen und eröffnet ein neues und breites Anwendungsfeld für Vitamin E.

Die Erfindung betrifft somit die Verwendung von Vitamin E zur Herstellung eines Arzneimittels zur Verstärkung und Verbesserung der Wirkung oral verabreichten 1,4-Dihydro-2,6-dimethyl-4-(2'-nitrophenyl-)pyridin-3,5-dicarbonsäure-dimethylesters (Nifedipins).

Die Erfindung betrifft auch Mittel zur Behandlung von Herzerkrankungen im Wege der oralen Verabreichung des Wirkstoffs, gekennzeichnet durch einen Gehalt an Vitamin E im Bereich von 200 bis 800 mg pro Verabreichungseinheit, 1,4-Dihydro-2,6-dimethyl-4-(2'-nitrophenyl)-pyridin-3,5-dicarbonsäure-dimethylester (Nifedipin) sowie gegebenenfalls weiteren Wirkstoffen sowie üblichen Träger- und Hilfsstoffen.

Die Erfindung betrifft letztlich Mittel zur Behandlung von Herzerkrankungen im Wege der Verstärkung und Verbesserung der Wirkung eines oral verabreichten, koronar wirksamen gefäßerweiternden Mittels, gekennzeichnet durch einen Gehalt an Vitamin E im Bereich von 80 bis 500 mg pro Verabreichungseinheit, 1,4-Dihydro-2,6-dimethyl-4-(2'-nitrophenyl-) pyridin-3,5-dicarbonsäure-dimethylester (Nifedipin) sowie gegebenenfalls weiteren Wirkstoffen und üblichen Träger- und Hilfsstoffen.

Erfindungsgemäß bevorzugt ist die Verwendung von Vitamin E zur Verstärkung und Verbesserung der Wirkung in Kapselform verabreichten Nifedipins.

Im Rahmen der erfindungsgemäßen Verwendung wird Vitamin E in einer Menge von 200 bis 800 mg, bevorzugt von 300 bis 600 mg, pro Kapsel eingesetzt.

Die erfindungsgemäßen Mittel zur Behandlung von Herzerkrankungen im Wege der oralen Verabreichung der Wirkstoffe sind bevorzugt Kapseln, die außer Nifedipin Vitamin E in einer Menge von 200 bis 800 mg pro Verabreichungseinheit, vorzugsweise von 300 bis 600 mg, besonders bevorzugt von 400 bis 500 mg, enthalten.

Die erfindungsgemäßen Mittel zur Behandlung von Herzerkrankungen im Wege der Verstärkung und Verbesserung der Wirkung eines oral verabreichten, koronar wirksamen gefäßerweiternden Mittels sind ebenfalls bevorzugt Kapseln, deren Gehalt an Vitamin E im Bereich von 80 bis 500 mg pro Verabreichungseinheit, vorzugsweise im Bereich von 150 bis 400 mg, besonders bevorzugt im Bereich von 200 bis 400 mg pro Verabreichungseinheit, liegt.

Das verwendete Vitamin E kann natürlichen oder synthetischen Ursprungs sein.

Als Vitamin E kann sowohl natürliches Vitamin E in Form von D-alpha-Tocopherol und seinen Konzentraten sowie die entsprechenden Ester verwendet werden. Bevorzugte Ester sind die Acetate. Weiterhin werden auch synthetisches D,L-alpha-Tocopherol bzw. sein Acetat eingesetzt.

Die erfindungsgemäßen Mittel enthalten außer dem Nifedipin und Vitamin E übliche Träger- und Hilfsstoffe. Da Vitamin E bei üblichen Temperaturen flüssig ist, bieten sich hierfür als Applikationsformen insbesondere Kapseln und vorzugsweise Weichgelatinekapseln an. Die übrigen Wirkstoffe werden in Vitamin E sowie gewünschtenfalls in einem dünnflüssigen Neutralöl und einem Lösungsvermittler wie Tween® in an sich bekannter Weise in Weichgelatinekapseln eingebracht. Hierbei können insbesondere die Standardrezepturen der Firma Scherer, Eberbach, zur Anwendung kommen.

Als weitere Träger- und Hilfsstoffe kommen Milchzucker, Polyethylenglykole, Kieselsäure und deren Derivate, und Emulgatoren in Frage.

Die erfindungsgemäßen Mittel können zusätzlich noch Vitamin A, und/oder ein oder mehrere Vitamine der B-Reihe und/oder B-Rezeptorenblocker und/oder Emulgatoren und/oder Neutralöle enthalten.

Es wird erfindungsgemäß bei der Herstellung der Kapseln Neutralöl zur Verdünnung verwendet.

Als Neutralöl zur Herstellung der Kapseln kann Sojaöl, bzw. hydriertes Sojaöl, Erdnußöl, Olivenöl, Triglyceride etc., verwendet werden.

Es können weitere Zusätze wie Vitamin A oder ein bzw. mehrere Vitamine der B-Reihe zugesetzt werden. Man kann auch β-Rezeptorenblocker zusetzen.

Durch den Zusatz von Nifedipin kann die Durchblutung des Herzens gesteigert werden. Eine derartige Kombination ist insbesondere zur vorbeugenden Behandlung herzinfarktgefährdeter Patienten geeignet.

Die neuen Mittel zur Behandlung von Herzerkrankungen sind besonders geeignet zur schnellen Linderung und Beseitigung von Schmerzen. Sie führen zu einer Stärkung des Herzmuskels bzw. der Koronargefäße. Sie bewirken dadurch auch bei längerer Anwendung eine Erhöhung der Belastbarkeit des Herzens. Dies kann bis zu einer Steigerung der Leistungsfähigkeit der Patienten führen.

Die erfindungsgemäßen Mittel sind somit besonders geeignet zur Therapie und Prophylaxe koronarer Durchblutungsstörungen. Weiterhin angezeigt sind sie bei Koronarinsuffizienz und zur Vorbeugung von Angina Pectoris.

In den nachfolgenden Beispielen sind typische Wirkstoffkombinationen und Dosierungen zusammengestellt.

**Beispiel 1**

Eine Kapsel enthält:
10 mg Nifedipin
400 mg DL-alpha-Tocopherolacetat
8.000 I.E. Vitamin-A-Palmitat
200 mg Sojaöl

**Beispiel 2**

Eine Kapsel enthält anstelle von
400 mg DL-alpha-Tocopherolacetat:

400 mg D-alpha-Tocopherolacetat,
die weitere Zusammensetzung entspricht Beispiel 1.

**Beispiel 3**

Eine Kapsel enthält:
20,0 mg Nifedipin
500 mg D-alpha-Tocopherolacetat
1.000 mg I.E. Vitamin A-Acetat
220 mg Sojaöl

**Beispiel 4**

Kapsel gemäß Beispiel 3, jedoch ohne Vitamin A.

**Beispiel 5**

Kapsel gemäß Beispiel 3, jedoch mit 6 mg β-Carotin anstelle von Vitamin-A-Acetat

**Ansprüche**

1. Verwendung von Vitamin E zur Herstellung eines Arzneimittels zur Verstärkung und Verbesserung der Wirkung oral verabreichten 1,4-Dihydro-2,6-dimethyl-4-(2'-nitrophenyl-)pyridin-3,5-dicarbonsäure-dimethylesters (Nifedipins).

2. Verwendung nach Anspruch 1 zur Herstellung eines Arzneimittels zur Verstärkung und Verbesserung der Wirkung in Kapselform verabreichten Nifedipins.

3. Verwendung nach einem oder mehreren der Ansprüche 1 und 2, wobei Vitamin E in Form von D-alpha-Tocopherol und/oder D,L-alpha-Tocopherol und/oder eines seiner Ester natürlichen oder synthetischen Ursprungs eingesetzt wird.

4. Verwendung nach einem oder mehreren der Ansprüche 1 bis 3, wobei Vitamin E in einer Menge von 200 bis 800 mg, bevorzugt von 300 bis 600 mg, pro Kapsel eingesetzt wird.

5. Mittel zur Behandlung von Herzerkrankungen im Wege der oralen Verabreichung des Wirkstoffs, gekennzeichnet durch einen Gehalt an Vitamin E im Bereich von 200 bis 800 mg pro Verabreichungseinheit, 1,4-Dihydro-2,6-dimethyl-4-(2'-nitrophenyl-)pyridin-3,5-dicarbonsäure-dimethylester (Nifedipin) sowie gegebenenfalls weiteren Wirkstoffen sowie üblichen Träger- und Hilfsstoffen.

6. Mittel nach Anspruch 5 in Form von Kapseln.

7. Mittel nach einem oder mehreren der Ansprüche 5 und 6, worin der Gehalt an Vitamin E im Bereich von 300 bis 600 mg, bevorzugt von 400 bis 500 mg, pro Verabreichungseinheit liegt.

8. Mittel nach einem oder mehreren der Ansprüche 5 bis 7, dadurch gekennzeichnet, daß sie zusätzlich Vitamin A und/oder ein oder mehrere Vitamin(e) der B-Reihe und/oder β-Rezeptorenblocker und/oder Emulgatoren und/oder Neutralöle und/oder Träger und/oder Hilfsstoffe enthalten.

9. Mittel nach Anspruch 8, dadurch gekennzeichnet, daß die Neutralöle Sojaöl, Erdnußöl, Olivenöl oder Gemische derselben sind.

10. Mittel zur Behandlung von Herzerkrankungen im Wege der Verstärkung und Verbesserung der Wirkung eines oral verabreichten, koronar wirksamen gefäßerweiternden Mittels, gekennzeichnet durch einen Gehalt an Vitamin E im Bereich von 80 bis 500 mg pro Verabreichungseinheit, 1`4-Dihydro-2,6-dimethyl-4-(2'-nitrophenyl-)pyridin-3,5-dicarbonsäure-dimethylester (Nifedipin) sowie gegebenenfalls weiteren Wirkstoffen und üblichen Träger- und Hilfsstoffen.

11. Mittel nach Anspruch 10, worin der Gehalt an Vitamin E im Bereich von 150 bis 400 mg, bevorzugt von 200 bis 400 mg, pro Verabreichungseinheit liegt.

12. Mittel nach einem oder mehreren der Ansprüche 5 bis 11, worin Vitamin E in Form von D-alpha-Tocopherol und/oder D,L-alpha-Tocopherol und/oder eines seiner Ester natürlichen oder synthetischen Ursprungs eingesetzt wird.

**Claims**

1. Use of vitamin E for the preparation of a medicament for enhancing and improving the effect of orally administered 1,4-dihydro-2,6-dimethyl-4-(2'-nitrophenyl)pyridine-3,5-dicarboxylic acid dimethylester (nifedipin).

2. Use according to claim 1 for the preparation of a medicament for enhancing and improving the effect of nifedipin administered in the form of capsules.

3. Use according to one or several of claims 1 and 2, wherein vitamin E is employed in the form of D-α-tocopherol and/or D,L-α-tocopherol and/or one of its esters of natural or synthetic origin.

4. Use according to one or several of claims 1 to 3, wherein vitamin E is employed in an amount of from 200 to 800 mg, and preferably from 300 to 600 mg, per capsule.

5. An agent for the treatment of cardiac diseases by way of an oral administration of the active ingredient, characterized by a vitamin E content of from 200 to 800 mg per dosage unit, 1,4-dihydro-2,6-dimethyl-4-(2'-nitrophenyl)-pyridine-3,5-dicarboxylic acid dimethylester (nifedipin) and optionally further active ingredients and conventional carriers and excipients.

6. The agent according to claim 5 in the form of capsules.

7. The agent according to one or several of claims 5 and 6, wherein the vitamin E content is from 300 to 600 mg, and preferably from 400 to 500 mg, per dosage unit.

8. The agent according to one or several of claims 5 to 7, characterized in that it additionally contains vitamin A and/or one or more vitamin(s) of the B series and/or β-adrenergic receptor blocking agents and/or emulsifiers and/or neutral oils and/or carriers and/or excipients.

9. The agent according to claim 8, characterized in that the neutral oils are soybean oil, peanut oil, olive oil or mixtures thereof.

10. An agent for the treatment of cardiac diseases by way of enhancing and improving the effect of orally administered coronary-active vasodilator, characterized by a vitamin E content within the range of from 80 to 500 mg per dosage unit, 1,4-dihydro-2,6-dimethyl-4-(2'-nitrophenyl)pyridine-3,5-dicarboxylic acid dimethylester (nifedipin) and optionally further active ingredients and conventional carriers and excipients.

11. The agent according to claim 10, wherein the vitamin E content is from 150 to 400 mg, and preferably from 200 to 400 mg, per dosage unit.

12. The agent according to one or several of claims 5 to 11, wherein vitamin E is employed in the form of D-α-tocopherol and/or D,L-α-tocopherol and/or one of its esters of natural or synthetic origin.

**Revendications**

1. Utilisation de la vitamine E pour la préparation d'un médicament destiné au renforcement et à l'amélioration de l'action du 1,4-dihydro-2,6-diméthyl-4-(2'-nitrophényl)pyridine-3,5-dicarboxylate de diméthyle (nifédipine) administré par voie orale.

2. Utilisation selon la revendication 1, pour la préparation d'un médicament destiné au renforcement et à l'amélioration de l'action de la nifédipine administrée sous la forme de capsules.

3. Utilisation selon l'une ou plusieurs des revendications 1 et 2, caractérisée en ce que la vitamine E est mise en oeuvre sous la forme de D-alpha-tocophérol et/ou de D,L-alpha-tocophérol et/ou d'un de ses esters d'origine naturelle ou synthétique.

4. Utilisation selon l'une ou plusieurs des revendications 1 à 3, caractérisée en ce que la vitamine A est mise en oeuvre selon une quantité allant de 200 à 800 mg, de préférence de 300 à 600 mg, par capsule.

5. Agent pour le traitement de maladies cardiaques, par administration de la substance active par voie orale, caractérisé en ce qu'il contient de 200 à 800 mg de vitamine E par unité administrée, du 1,4-dihydro-2,6-diméthyl-4(2'-nitrophényl)pyridine-3,5-dicarboxylate de diméthyle (nifédipine), ainsi qu'éventuellement d'autres substances actives, et des supports et adjuvants usuels.

6. Agent selon la revendication 5, sous la forme de capsules.

7. Agent selon l'une ou plusieurs des revendications 5 et 6, caractérisé en ce qu'il contient de 300 à 600 mg de vitamine E, de préférence de 400 à 500 mg, par unité administrée.

8. Agent selon l'une ou plusieurs des revendications 5 à 7, caractérisé en ce qu'il contient, en outre, de la vitamine A et/ou une ou plusieurs vitamines du groupe B, et/ou des β-bloquants, et/ou des émulsifiants, et/ou des huiles neutres, et/ou des supports, et/ou des adjuvants.

9. Agent selon la revendication 8, caractérisé en ce que les huiles neutres sont l'huile de soja, l'huile d'arachide, l'huile d'olive, ou des mélanges de ces huiles.

10. Agent pour le traitement de maladies cardiaques, pour le renforcement et l'amélioration de l'action d'un agent vasodilatateur efficace du point de vue coronarien, administré par voie orale, caractérisé en ce qu'il contient de 80 à 500 mg de vitamine E par unité administrée, de 1,4-dihydro-2,6-diméthyl-4-(2'-nitrophényl)-pyridine-3,5-dicarboxylate de diméthyle (nifédipine), ainsi qu'éventuellement d'autres substances actives et des véhicules et adjuvants usuels.

11. Agent selon la revendication 10, caractérisé en ce qu'il contient de 150 à 400 mg de vitamine E, de préférence de 200 à 400 mg, par unité administrée.

12. Agent selon l'une ou plusieurs des revendications 5 à 11, caractérisé en ce que la vitamine E est mise en oeuvre sous la forme de D-alpha-tocophérol et/ou de D,L-alpha-tocophérol et/ou d'un de ses esters d'origine naturelle ou synthétique.